# EUROPEAN PATENT SPECIFICATION

(11) **EP 3 087 852 B1**
(45) Date of publication and mention of the grant of the patent: **07.11.2018**
(21) Application number: 16165614.5
(22) Date of filing: 15.04.2016
(51) Int. Cl.: A24B 13/00

(54) **ORAL POUCHED PRODUCT HAVING A RECTANGULAR SHAPE**
ORALES PRODUKT IN BEUTELFORM MIT RECHTECKIGER FORM
PRODUIT EN SACHET ORAL PRÉSENTANT UNE FORME RECTANGULAIRE

(30) Priority: 17.04.2015 EP 15164065
(43) Date of publication of application: 02.11.2016
(73) Proprietor: Swedish Match North Europe AB, 118 85 Stockholm (SE)
(72) Inventor: HASSLER, Thord, 252 20 Helsingborg (SE); ASPGREN, Thom, 422 57 Hisings Backa (SE); WERDINIUS, Christian, 417 20 Göteborg (SE)
(74) Representative: Valea AB

(56) References cited:
- WO-A1-2010/104464
- WO-A1-2011/129883
- WO-A1-2012/134380
- WO-A1-2013/174991
- US-A1- 2008 202 536
- US-A1- 2014 261 472
- Anonymous: "Snubie.com Snus Reviews, News, and Information.: Camel "SNUS" Mint - Review. 4 November 2014.", , 4 November 2014 (2014-11-04), XP055299769, Retrieved from the Internet: URL:http://chadizzy1.blogspot.nl/2014/11/c amel-mint-snus-review-4-november-2014.html [retrieved on 2016-09-05]
- "camel_snus_compare", , 26 October 2015 (2015-10-26), XP055223521, Retrieved from the Internet: URL:http://goodhealth.freeservers.com/Came l_SNUS_cparing_pouch_szie.jpg [retrieved on 2015-10-26] & Anonymous: "tobacco | Banal Leakage", , 22 December 2010 (2010-12-22), XP055223364, Retrieved from the Internet: URL:http://www.banalleakage.com/tag/tobacc o/ [retrieved on 2015-10-26]
- "The LAB Snus online - Portionssnus - Svenskt Snus - snus.swedishmatch.com", , 1 September 2009 (2009-09-01), XP055223391, Retrieved from the Internet: URL:http://snus.swedishmatch.com/sv/Produk ter/01-02-LabSeries/ [retrieved on 2015-10-26]
- Anonymous: "02 Strong, 21.6g, Portion Snus by Swedish Match - Portion", , 26 October 2015 (2015-10-26), XP055223400, Retrieved from the Internet: URL:http://www.northerner.com/the-lab-02-s trong-slim-portion.html [retrieved on 2015-10-26]
- Anonymous: "01 Original Portion", , 26 May 2012 (2012-05-26), XP055223394, Retrieved from the Internet: URL:http://blog.northerner.com/reviews/snu s-reviews/01-original-portion/ [retrieved on 2015-10-26]
- Anonymous: "The Mint Snuff Story - how Mint Snuff herbal chew was invented", , 26 October 2015 (2015-10-26), XP055223517, Retrieved from the Internet: URL:http://mintsnuff.com/mintsnuffstory.ht m [retrieved on 2015-10-26]
- Anonymous: "Zonnic Pouch | Niconovum AB", , 26 October 2013 (2013-10-26), XP055223512, Retrieved from the Internet: URL:http://www.niconovum.se/Products.aspx? id=8 [retrieved on 2015-10-26]

## Description

### TECHNICAL FIELD

The present invention relates to an oral pouched product, such as an oral pouched smokeless tobacco product, comprising a filling material, such as tobacco material, and a saliva-permeable pouch enclosing the filling material.

### BACKGROUND

Smokeless tobacco for oral use includes chewing tobacco, dry snuff and moist (wet) snuff. Generally, dry snuff has a moisture content of less than 10 wt% and moist snuff has a moisture content of above 40 wt%. Semi-dry products having between 10% to 40 wt% moisture content are also available.

Smokeless tobacco products for oral use are made from tobacco leaves, such as lamina and stem of the tobacco leaf. The material from roots and stalks are normally not utilized for production of smokeless tobacco compositions for oral use.

There are two types of moist snuff, the American type and the Scandinavian type which is also called snus. American-type moist snuff is commonly produced through a fermentation process of tobacco. Scandinavian-type moist snuff is commonly produced by using a heat-treatment process (pasteurization). The heat-treatment is carried out in order to degrade, destroy or denature at least a portion of the microorganisms in the tobacco preparation.

Both the American-type and the Scandinavian-type of moist snuff for oral use are available in loose form or portion-packed in a saliva-permeable, porous wrapper material forming a pouch. Pouched moist snuff, including snus, is typically used by the user by placing the pouch between the upper or lower gum and the lip or cheek and retaining it there for a limited period of time. The pouch material holds the tobacco in place while allowing saliva to pass into the interior of the pouched product and allowing flavours and nicotine to diffuse from the tobacco material into the user's mouth.

The pouch material is typically made of a nonwoven fleece (soft fabric) material, such as viscose (regenerated cellulose; viscose fibres are often referred to as viscose rayon or rayon), including an acrylic polymer that acts as binder in the nonwoven material and provides for heat-sealing of the pouches during manufacturing thereof. The viscose nonwoven material normally used for pouched smokeless tobacco products is similar to the fabric used in tea bags. Nonwovens are fabrics that are neither woven nor knitted. Methods for the manufacturing of nonwoven materials are commonly known in the art.

Pouched smokeless tobacco products may be produced by measuring portions of the smokeless tobacco composition and inserting the portions into a nonwoven tube. US 4,703,765 discloses a device for packaging precise amounts of finely divided tobacco products, such as snuff tobacco or the like, in a tubular packaging material into which snuff portions are injected via a fill tube. Downstream from the tube, welding means are positioned for transverse sealing of the packaging material, and also cutting means for severing the packaging material in the area of the transverse seal to thus form discrete or individual portion packages. EP 2428450 B1 relates to a snus dosing method, wherein a portion of tobacco is filled into a dosing chamber of a dosing device and then blown out of the dosing chamber by means of blow-out air to which water vapor has been added.

Pouched smokeless tobacco products may alternatively be produced by placing portions of moist snuff on a nonwoven web using a pouch packer machine in accordance with the device disclosed in US 6,135,120. This device comprises feeding means for feeding the tobacco material into pockets formed in a rotary portioning wheel for portioning the material into portions, at least one compression means for compressing the tobacco material portions, a unit for advancing a packaging material, such as a nonwoven web, in synchrony with the compressed portions, at least one discharge means for discharging the portions from the pockets to the packaging material, and a forming unit for forming individual portion packages (i.e. pouched smokeless tobacco products) from the discharged portions and the packaging material. At the intended point of discharge of the portions of to the packaging material, said packaging material has the form of a tape, the compression means being arranged to compress the portions in a direction which differs from the discharging and the feeding directions. The compression is preferably effected in a direction perpendicular to the discharging and the feeding directions. The compression may be effected in the axial direction of the portioning wheel whereas the feeding and discharging may be effected in the radial direction of said wheel. This technique is herein referred to as the "NYPS" technique.

The individual portions are sealed and cut apart thereby forming rectangular "pillow shaped" (or any other desired form) pouched products. Generally, each final pouched product includes parallel transverse seams with seals at opposite ends and a longitudinal seam with a seal orthogonal to the transverse seams/seals. The seals should be of sufficient strength to preserve the integrity of the pouched product during use while not disturbing the user's experience.

The organoleptic properties, such as texture, aroma, taste, shape and appearance, of the pouched smokeless tobacco product are of high importance to the user.

Oral pouched smokeless tobacco products are normally sized and configured to fit comfortably and discreetly in a user's mouth between the upper or lower gum and the lip. In general, oral pouched smokeless tobacco products have a generally rectangular shape. Some typical shapes (length x width) of commercially available oral pouched smokeless tobacco products are, for instance, 35 mm x 20 mm, 34/35 mm x 14 mm, 33/34 mm x 18 mm, and 27/28 mm x 14 mm. The thickness ("height") of the pouched product is normally within the range of from 5 to 7 mm. The total weight of commercially available oral pouched smokeless tobacco products are typically within the range from about 0.3 to about 3.5 g, such as from about 0.5 to 1.7 g, per pouched product.

US 2014/261472 A1 relates to a fiber-wrapped smokeless tobacco product which includes smokeless tobacco and a plurality of polymeric fibers surrounding the smokeless tobacco. Alternative shapes for the fiber-wrapped smokeless tobacco product are depicted.

US 2008/202536 A1 relates to an oral pouch flavor product with a fibrous flavored wrapper that encloses an inner filling material. Preferably, the fibrous flavored wrapper includes embedded flavorants. The fibrous flavored wrapper may also include a softening agent, colorants, and/or browning inhibitors. The inner material may include non-tobacco, plant material fibers and preferably has a low moisture content so as to prevent the need for preservatives or refrigeration.

WO 2011/129883 A1 relates to a pouch product including a pouch wrapper formed of a web having a longitudinal integrated fin and lap seal. The pouch wrapper contains a filling material including tobacco or non-tobacco material and optional additives. The longitudinal integrated fin and lap seal is formed on a forming collar incorporated in a pouching apparatus.

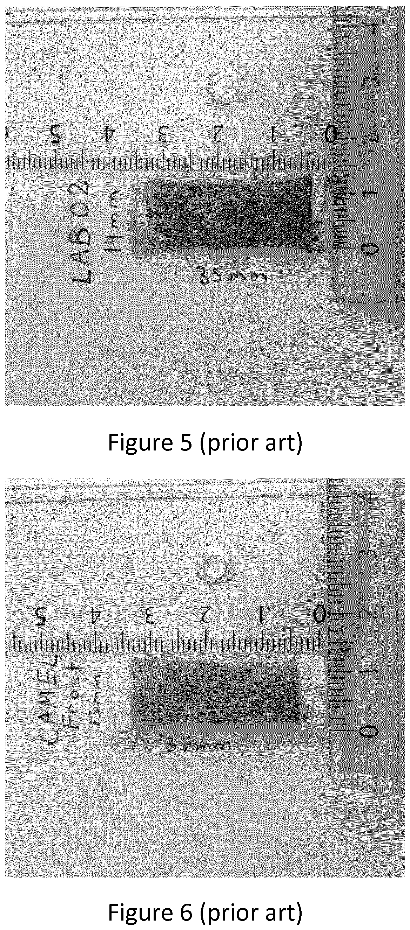

Examples of some present commercially available oral pouched snus products are shown in Figures 5 and 6.

For users having a small space between the upper (or lower) gum and the upper (or lower) lip, the oral pouched smokeless tobacco product may be visible, for instance, when the user smiles. Some users find this problematic and undesirable.

Moreover, users having this type of mouth geometry as well as other users may experience that the pouched product rubs on the interface between gum and teeth which may cause pain and/or gum recession.

It is generally desirable to provide oral pouched smokeless tobacco products with rapid release of flavour and nicotine to reduce nicotine craving and give an initial strong flavour experience. In particular, previous smokers which are used to quick nicotine stimulation from cigarettes desire oral pouched smokeless tobacco products with rapid nicotine release.

As mentioned above, saliva passes into the interior of the pouched product and allows flavours and nicotine to diffuse from the tobacco material into the user's mouth.

The user's experience may be affected by the initial moisture content of the pouched product when put in the user's mouth and the saliva uptake (rate of uptake and amount of saliva taken up) by the pouched product.

Oral pouched smokeless tobacco products may be post-moisturized after pouch formation or not post-moisturized after pouch formation which herein is referred to as non-post-moisturized. Post-moisturized pouched products may be produced by spraying water on the pouched smokeless tobacco product before packaging the pouched products in cans. Post-moisturized pouches are sometimes referred to as "original snus". Non-post-moisturized pouched products are sometimes referred to as "white snus" and are by some users considered to have a more appealing appearance. The moisture content of the final oral pouched smokeless tobacco product comprising moist or semi-dry snuff is normally within the range of from 25 to 55% w/w based on the weight of the pouched product (i.e. the total weight of snuff and pouch material).

Saliva is secreted by major and minor salivary glands located in the oral cavity. The major salivary glands are the parotid glands, the submandibular glands and the sublingual glands. There are also about 800 to 1000 minor salivary glands located in the mucosa throughout the oral cavity, such as in the tissue of the buccal and labial mucosa.

When the oral pouched smokeless tobacco product has initial high moisture content and/or a large amount of saliva is taken up by the pouched product, the user may experience that the pouched product becomes too runny (flowing) even though the taste may be satisfactory. Thus, the user may experience an unpleasant excessive amount of saliva/water in the mouth when the pouched product has been used by the user a period of time, such as after about 15-40 minutes.

Thus, there is a need for an improved oral pouched smokeless tobacco product providing a satisfactory taste experience and nicotine release and configured to fit comfortably and discreetly in a user's mouth irrespective of the user's mouth geometry while avoiding the feeling of runniness upon use by the user.

### SUMMARY OF THE INVENTION

An object of the present invention is to alleviate one or more of the problems discussed above, and to provide advantages and aspects not provided by hitherto known oral pouched products, in particular oral pouched smokeless tobacco products.

According to a first aspect, there is provided an oral pouched smokeless tobacco product comprising a filling material and a saliva-permeable pouch enclosing the filling material, the product having a rectangular shape with a maximum length and a maximum width, characterized in that the maximum width of the product is within the range of from 5 mm to 10 mm, the maximum length of the product is within the range of from 25 mm to 40 mm, the ratio of maximum length to maximum width is within the range of from 3 to 6, the oral pouched smokeless tobacco product has a weight within the range of from 0.3 g to 0.7 g, the oral pouched smokeless tobacco product has a moisture content within the range of 40 to 60% w/w based on the total weight of the oral pouched smokeless tobacco product, and the product has a thickness in a direction perpendicular to the width of the product, said thickness being within the range of from 2 to 8 mm.

It is believed that the comparatively narrow width and the specified length-to-width ratio of the oral pouched product as disclosed herein, such as the oral pouched smokeless tobacco product as disclosed herein, may mitigate pressure on the gum/teeth interface upon use of the oral pouched product, thereby reducing the risk for uncomfortable rubbing and gum recession.

The oral pouched product as disclosed herein may fit discreetly and comfortably also in the mouth of users having a small space between the upper (or lower) gum and the upper (or lower) lip.

The oral pouched product as disclosed herein may also be more prone to stay in place in the mouth upon usage thereof.

As disclosed herein, the oral pouched product may be an oral pouched smokeless tobacco product and the filling material may then comprise tobacco material.

As disclosed herein, the oral pouched product may be a non-post-moisturized oral pouched smokeless tobacco product.

As disclosed herein, the oral pouched product may be a non-post-moisturized oral pouched smokeless tobacco product having moisture content within the range of from 45 to 55% w/w, such as within the range of from 50 to 53% w/w, based on the total weight of the product. This oral pouched smokeless tobacco product provides a satisfactory taste experience while avoiding the feeling of runniness upon use by the user. The oral pouched smokeless tobacco product has also been found to provide a satisfactory perceived nicotine delivery.

As disclosed herein, the oral pouched product may be a non-post-moisturized oral pouched smokeless tobacco product having moisture content within the range of from 45 to 55% w/w, such as within the range of from 50 to 53% w/w, based on the total weight of the product and the pouch may contain a longitudinal fin seam extending along the length of the product, said longitudinal seam having a width within the range of from 0.5 mm to 2 mm, such as from 0.5 mm to 1 mm.

As disclosed herein, the oral pouched product may be a non-post-moisturized oral pouched smokeless tobacco product having moisture content within the range of from 45 to 55% w/w, such as within the range of from 50 to 53% w/w, based on the total weight of the product and the pouch may contain at least one transverse fin seam extending along the width of the product, said at least one transverse seam having a width within the range of from 0.5 mm to 2mm, such as from 0.5 mm to 1 mm.

### BRIEF DESCRIPTION OF THE DRAWINGS

Figure 1a and Figure 1b show an embodiment of the oral pouched product as disclosed herein and how to determine the length (L), width (W) and thickness (T) thereof. As shown in Fig. 1a, the length (L) of the oral pouched product as disclosed herein is the maximum length of the oral pouched product and the width (W) of the oral pouched product as disclosed herein is the maximum width of the oral pouched product.
Figure 2a illustrates a lap seal, Figure 2b illustrates a fin seam and Figure 2c illustrates a combined fin-and-lap seal.
Figure 3a illustrates where to cut the product in order to measure the transverse waist circumference. Figure 3b illustrates where to cut the product in order to measure the longitudinal circumference.
Figure 4 illustrates the sensory characteristics experienced by a panel of users when using oral pouched smokeless tobacco products with varying length-to-width ratios.
Figure 5 illustrates the maximum length and the maximum width of the commercially available pouched snus product "LaB 02" (prior art).
Figure 6 illustrates the maximum length and the maximum width of the commercially available pouched snus product "Camel Frost" (prior art).

### DETAILED DESCRIPTION

By "tobacco" is meant any part, e.g., leaves, stems, and stalks, of any member of the genus Nicotiana. The tobacco may be whole, shredded, threshed, cut, ground, cured, aged, fermented, or treated otherwise, e.g., granulated or encapsulated.

The term "tobacco material" is used herein for tobacco leaves or parts of leaves, such as lamina and stem. The leaves and parts of leaves may be finely divided (disintegrated), such as ground, cut, shredded or threshed, and the parts of leaves may be blended in defined proportions in the tobacco material.

"Oral" and "oral use" is in all contexts used herein as a description for use in the oral cavity of a human, such as buccal placement.

The terms "oral pouched products" and "oral pouched snuff products" as used herein include oral pouched non-tobacco snuff products, which may be nicotine-containing or nicotine-free, as well as oral pouched tobacco-containing snuff products (also called oral pouched smokeless tobacco products).

As used herein the terms "pouched snuff product for oral use" or "oral pouched snuff product" refer to a portion of smokeless tobacco or tobacco-free filling material, which may be nicotine-containing or nicotine-free as described herein, packed in a saliva-permeable pouch material intended for oral use.

As used herein "pouched smokeless tobacco product for oral use" or "oral pouched smokeless tobacco product" refers to a portion of smokeless tobacco packed in a saliva-permeable pouch material intended for oral use.

As used herein, the term "moisture content" refers to the total amount of oven volatile ingredients, such as water and other oven volatiles (e.g. propylene glycol) in the preparation, composition or product referred to. The moisture content is given herein as percent by weight (wt%) of the total weight of the preparation, composition or product referred to.

The moisture content as referred to herein may be determined by using a method based on literature references Federal Register/ vol.74, no. 4/712-719/Wednesday, January 7, 2009/Notices "Total moisture determination" and AOAC (Association of Official Analytical Chemics), Official Methods of Analysis 966.02: "Moisture in Tobacco" (1990), Fifth Edition, K. Helrich (ed). In this method, the moisture content is determined gravimetrically by taking 2.5±0.25 g sample and weighing the sample before evaporation of moisture and after completion of dehydration. Mettler Toledo's Moisture Analyzer HB43, a balance with halogen heating technology, is used (instead of an oven and a balance as in the mentioned literature references) in the experiments described herein. The sample is heated to 105°C (instead of 99.5±0.5°C as in the mentioned literature references). The measurement is stopped when the weight change is less than 1 mg during a 90 seconds time frame. The moisture content as weight percent of the sample is then calculated automatically by the Moisture Analyzer HB43.

The term "additional ingredient" as used herein denotes substances other than tobacco material, salt (e.g. sodium chloride, potassium chloride, magnesium chloride, calcium chloride and any combinations thereof), pH adjuster (e.g. sodium hydroxide, potassium hydroxide, potassium carbonate, sodium carbonate or sodium bicarbonate) and water.

"Flavour" or "flavouring agent" is used herein for a substance used to influence the aroma and/or taste of the smokeless tobacco product, including, but not limited to, essential oils, single flavour compounds, compounded flavourings, and extracts.

As used herein "finely divided" means an average particle size of less than 2 mm. The particles of the finely divided tobacco material may be sized to pass through a screen of about 10 (US) mesh, i.e. sieve size 2.0 mm, or 18 (US) mesh, i.e. sieve size 1.0 mm.

As used herein "% w/w" or "wt %" or "weight %" refers to weight percent of the ingredient referred to of the total weight of the preparation, composition or product referred to.

As used herein, reference to "dry weight percent" refers to weight percent of the ingredient referred to on the basis of the total weight of dry ingredients, i.e. all ingredients of the preparation, composition or product referred to excluding moisture content.

According to a first aspect of the invention, there is provided an oral pouched (snuff) product according to claim 1, namely, a smokeless tobacco product comprising a filling material and a saliva-permeable pouch enclosing the filling material, the product having a rectangular shape with a maximum length and a maximum width, characterized in that the maximum width of the product is within the range of from 5 mm to 10 mm, the maximum length of the product is within the range of from 25 mm to 40 mm, the ratio of maximum length to maximum width is within the range of from 3 to 6, the oral pouched smokeless tobacco product has a weight within the range of from 0.3 g to 0.7 g, the oral pouched smokeless tobacco product has a moisture content within the range of 40 to 60% w/w based on the total weight of the oral pouched smokeless tobacco product, and the product has a thickness in a direction perpendicular to the width of the product, said thickness being within the range of from 2 to 8 mm.

As disclosed herein, the oral pouched product may be an oral pouched smokeless tobacco product and the filling material may then comprise tobacco material, in particular tobacco material in the form of moist snuff or snus.

However, the oral pouched product may alternatively or additionally contain any other botanical filling material, such as any non-tobacco plant fibres.

The oral pouched product as disclosed herein may be an oral pouched non-tobacco snuff product.

The oral pouched product as disclosed herein may be an oral pouched non-tobacco nicotine-free snuff product comprising a botanical filling material, such as one or more non-tobacco plant fibers. Examples of non-tobacco plant fibers are maize fibers, oat fibers, tomato fibers, barley fibers, rye fibers, sugar beet fibers, buck wheat fibers, potato fibers, apple fibers, cocoa fibers, bamboo fibers and citrus fibers.

The oral pouched product as disclosed herein may be an oral pouched non-tobacco nicotine-containing snuff product. The filling material may then be a particulate material comprising nicotine or a salt thereof and one or more fillers, such as polysaccharides (e.g. maltitol and mannitol) and/or microcrystalline cellulose.

An oral pouched smokeless tobacco product generally includes a tobacco composition comprising divided (e.g. ground or cut) tobacco material, water, salt (e.g. sodium chloride, potassium chloride, magnesium chloride, calcium chloride or any combinations thereof), pH adjuster (e.g. sodium carbonate, sodium hydroxide, potassium hydroxide, potassium carbonate, sodium carbonate , sodium bicarbonate or magnesium carbonate) and optionally one or more additional ingredients, such as flavouring agents, cooling agents, heating agents, sweetening agents, colorants, humectants (e.g. glycerol or propylene glycol), antioxidants, preservatives (e.g. as potassium sorbate), binders, fillers, non-tobacco plant fibers and/or disintegration aids. The smokeless tobacco composition may be a moist snuff composition, such as a snus composition.

Typically, the amount of tobacco material in the smokeless tobacco composition is within the range of from about 50 to about 80% w/w based on dry weight of the smokeless tobacco composition. The tobacco material is typically finely divided, such as cut (shredded) or ground tobacco material, in granulated form or in powder form, i.e. tobacco flour, for instance having an average particle size of about 1 mm to about 2 mm. The tobacco material may be cured (aged) tobacco material.

Salt, such as sodium chloride, potassium chloride, magnesium chloride, calcium chloride and any combinations thereof, is added mainly for its effect on taste but it also has a preservative action which contributes to improved shelf life of the product. Salt, such as sodium chloride lowers the water activity of the products, thus preventing microorganisms from growing. The natural occurrence of sodium chloride in tobacco material is normally below 2% w/w, typically below 1% w/w, based on dry weight of the tobacco material. Normally, the amount of added salt in the smokeless tobacco composition is within the range of from about 0.5 to about 10% w/w based on dry weight of the tobacco composition.

pH adjusters, such as sodium carbonate, are added to bring the pH value to the slightly alkaline side, such as about pH 7.5 to 8.5. Sodium carbonate may also be used to give the products their characteristic aroma profile. Typically, the amount of pH adjuster in the smokeless tobacco composition is less than about 7% w/w, such as within the range of from 3 to 5% w/w, based on dry weight of the tobacco composition.

Humectants, such as propylene glycol or glycerol, may also be added to protect the product from drying out and may also have a preservative effect since the water activity of the product will be lowered, thereby preventing microorganisms from growing. Normally, the amount of humectant in the smokeless tobacco composition is within the range of from about 5 to about 15% w/w based on dry weight of the tobacco composition.

Flavours used are generally natural or nature identical compounds that comply with food regulations. Flavours are usually dissolved in ethanol when added.

In addition, the smokeless tobacco composition may optionally comprise other botanical filling material, such as any non-tobacco plant fiber. Examples of non-tobacco plant fibers are maize fibers, oat fibers, tomato fibers, barley fibers, rye fibers, sugar beet fibers, buck wheat fibers, potato fibers, apple fibers, cocoa fibers, bamboo fibers and citrus fibers. The amount of non-tobacco plant fiber material, such as bamboo fibers, in the smokeless tobacco composition may be within the range of from about 1 to about 60% w/w, such as from about 2 to about 20% w/w, based on dry weight of the smokeless tobacco composition.

The pouched product as disclosed herein are intended for use in the oral cavity, such as buccal placement (e.g. by placing the pouched product between the upper or lower gum and the lip or cheek), and may therefore be referred to as oral pouched product or portion-packed (pouched) product for oral use. The oral pouched product is sized and configured to fit comfortably and discreetly in a user's mouth between the upper or lower gum and the lip or cheek.

The oral pouched product as disclosed herein may be a post-moisturized or non-post-moisturized oral pouched smokeless tobacco product. The moisture content of the oral pouched smokeless tobacco product as disclosed herein may be within the range of from 40 to 60% w/w or from 40 to 55% w/w or from 45 to 55% w/w or from 50 to 55% w/w, based on the total weight of the product.

The oral pouched product as disclosed herein may be a non-post-moisturized oral pouched smokeless tobacco product having moisture content within the range of from 40 to 60% w/w or from 40 to 55% w/w or from 45 to 55% w/w or from 50 to 55% w/w or from 50 to 53% w/w, based on the total weight of the product.

In particular, the oral pouched product as disclosed herein may be a non-post-moisturized oral pouched smokeless tobacco product having moisture content within the range of 45 to 55% w/w, based on the total weight of the product. In such case, the dry weight of the oral pouched smokeless tobacco product may be within the range of from 0.14 g to 0.4 g.

The maximum width (W) of the oral pouched product as disclosed herein is within the range of claim 1, such as within the range of from 5 mm to 9 mm or from 7 mm to 9 mm.

Thus, the maximum width (W) of the oral pouched product as disclosed herein may be less than 10 mm, such as within the range of from from 5 mm to 9.5 mm or 7 mm to 9.5 mm.

The ratio of maximum length to maximum width (L/W) of the oral pouched product as disclosed herein is within the range of from 3 to 6, such as within the range of from 3 to 5 or from 3.5 to 4.5.

The maximum length (L) of the oral pouched product as disclosed herein is within the range of from 25 mm to 40 mm.

The oral pouched product as disclosed herein may have a maximum length within the range of from 25 mm to 40 mm and a maximum width within the range of from 5 mm to 9.5 mm as long as the ratio of maximum length to maximum width is within the range of from 3 to 6.

The oral pouched product as disclosed herein has a rectangular shape.

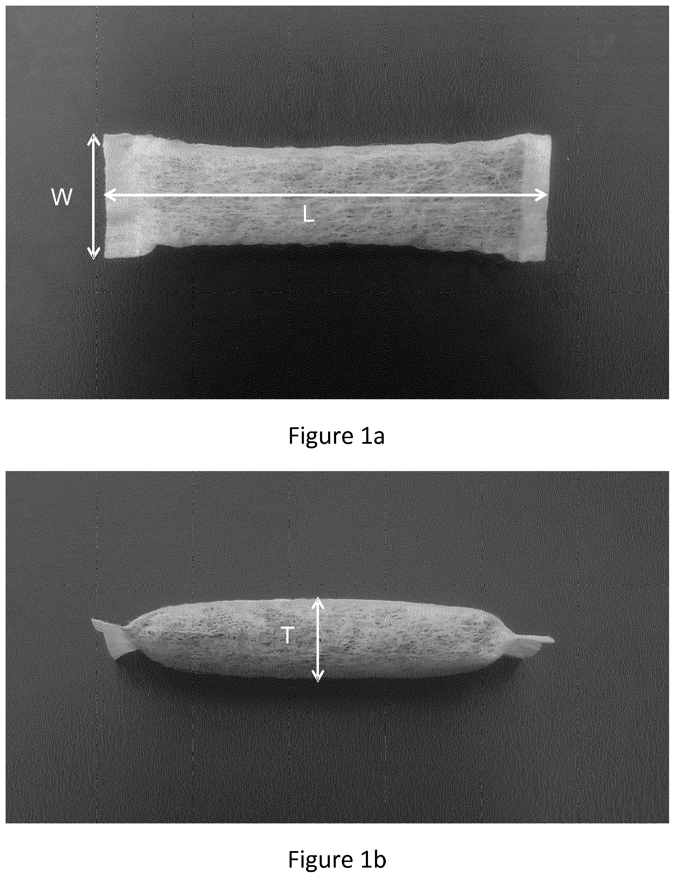

Figure 1a illustrates how the maximum width (W) and the maximum length (L) of the product are measured.

Figure 1b illustrates how the thickness (T) of the product is measured. The thickness is measured as the height of the product (in a non-compressed state) at the centre of the maximum length of the product, i.e. at half the maximum length of the product. The centre of the maximum length of the product is herein referred to as the waist of the product. The thickness of the product is herein therefore also referred to as "thickness at waist" of the oral pouched product.

The thickness (T) at half the maximum length of the oral pouched product as disclosed herein is within the range of from 2 to 8 mm, such as within the range of from 3 to 7 mm or from 4 to 6 mm.

The total weight of the oral pouched product (including filling material and pouch) is within the range of from 0.3 g to 0.7 g.

The dry weight of the oral pouched product (including filling material and pouch) may be within the range of from 0.2 g to 0.4 g. The dry weight of the oral pouched product is the total weight of the oral pouched product excluding the moisture content of the oral pouched product.

The ratio between length circumference and transverse waist circumference (LC-to-TWC ratio), as defined in Example 1 hereinafter, of the pouch of the oral pouched product as disclosed herein may be within the range of from 3 to 6.

The thickness at waist and the width at waist of the oral pouched product as disclosed herein may be substantially the same. Thus, the width at waist of the oral pouched product as disclosed herein may be within the range of from 2 to 8 mm, such as within the range of from 3 to 7 mm or from 4 to 6 mm.

In particular, the thickness of the product at half the maximum length of the product may be within the range of from 4 mm to 6 mm and the width at half the maximum length of the product may be within the range of from 4 mm to 6 mm.The ratio between the maximum length (L) of the product and the width at the waist of the oral pouched product as disclosed herein may be within the range of from 5 to 12.

The pouch of the oral pouched product may be made of any suitable saliva-permeable (and preferably non-dissolvable) pouch material, such as non-woven, woven or knitted materials made from fibers of cellulose (e.g. cotton), regenerated cellulose (e.g. viscose) or synthetic polymers (e.g. aliphatic polyesters or aliphatic polyamides).

The pouch material may be a nonwoven material of viscose (rayon staple fibres).

The pouch material may comprise additional ingredients, such as flavouring agents and/or colorants.

As used herein, the term "seam" refers to those parts of the pouch material which are brought into contact with one another in order to form the pouch. The seam further comprises a seal. The seal may be narrower in width than the seam. Alternatively, the entire width of the seam contains the seal (i.e. the seam and seal have the same width).

The longitudinal seam/seals of the pouch may be a lap seam/seal, a fin seam/seal or a combined lap-and-fin seam/seal.

The longitudinal seal, such as longitudinal fin seal, may be narrower in width than the longitudinal seam.

Alternatively, the width of the longitudinal seal, such as longitudinal fin seal, may correspond in width to the width of the longitudinal seam.

Still alternatively, the pouch material may be formed in tubular configuration such that a longitudinal seam/seal is not necessary. In such case, the pouch of the oral pouched product as disclosed herein may include at least one transverse seam/seal.

The longitudinal seam/seal of the pouch may be a symmetrically placed seam/seal or an asymmetrically placed seam/seal.

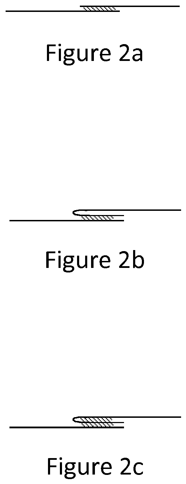

A lap seam/seal is formed by bringing an outer surface portion of the pouch material and an inner surface portion of the pouch material into a superposed relation. A lap seal is illustrated in Figure 2a.

A fin seam/seal is formed by bringing inner surface portions of the pouch material into a superposed relation. A fin seal is illustrated in Figure 2b.

A combined lap-and-fin seam/seal is formed by first bringing inner surface portions of the pouch material into a superposed relation, optionally sealing to form a fin seal, and then lap sealing the fin seam/seal to an outer surface portion of the pouch material. A combined lap-and-fin seal is illustrated in Figure 2c.

In this context, "inner surface" of the pouch material refers to the surface of the pouch material that will form the interior of the final pouch, i.e. the side of the pouch material that will face the filling material enclosed in the pouch. "Outer surface" of the pouch material refers to the surface of the pouch material that will form the exterior of the final pouch.

For illustrative purposes, Figures 2a, 2b and 2c show seals held together by adhesive illustrated by the dashed regions. If any one of these seals instead is created by, for instance heat melt-welding or ultrasonic welding, portions of the pouch material will form a solid-state weld (i.e. no dashed region exists).

The transverse seams/seals of the pouch may be fin seams/seals.

Each of the transverse fin seals may correspond in width to the width of the respective transverse fin seam.

Fin seals may have a higher strength than lap seals.

The seals may be provided by any known suitable sealing method, such as by sewing, by applying an adhesive and/or by using a welding method selected from the groups consisting of heat melt-welding (heat and pressure), high frequency welding (high frequency electromagnetic waves and pressure) and ultrasonic welding (high frequency ultrasonic acoustic vibrations, such as about 15 kHz to 70 kHz, and pressure).

When a welding method is used, a binder may be used in the pouch material to facilitate sealing of the material. The binder may be any suitable adhesive material, and suitable binders will be known to the skilled person. For example, thermoplastic binders based on polyacrylates can be used as suitable polymer binders. Viscose nonwoven including an acrylic polymer is normally used for pouched smokeless tobacco products.

Heat melt-welding is commonly used today in the production of oral pouched smokeless tobacco products and this method may be used to create the transverse seal(s) and/or the longitudinal seal, if present, of the oral pouched product as disclosed herein. Fin seals created by heat melt-welding may be within the range of from about 2 mm to about 5 mm in width. This means that fin seams comprising fin seals created by heat melt-welding may be within the range of from about 2 mm to about 5 mm in width.

Ultrasonic welding may be used to generate seals that are narrower in width (smaller) and more precise. Such seals are therefore tidier, more visually appealing and more discrete. They may also be more comfortable in the mouth of the user. Narrower seams/seals have the further advantage that the amount of pouch material required is reduced. Fin seals created by ultrasonic welding may be within the range of from about 0.1 mm to about 2 mm in width, such as within the range of from 0.1 mm to about 1 mm or 0.5 mm to about 1 mm in width. By using ultrasonic welding to create fin seals, the fin seams may be within the range of from about 0.5 mm to about 2 mm in width, such as from about 0.5 mm to 1 mm in width.

Ultrasonic welding may be used to create the transverse seal(s) and/or the longitudinal seal, if present, of the oral pouched product as disclosed herein. In such case, the transverse seams(s) and/or the longitudinal seam, if present, may be within the range of from about 0.5 mm to about 2 mm in width, such as within the range of from 0.5 mm to 1 mm in width.

Alternatively, heat melt-welding may be used to create the transverse seal(s) and ultrasonic welding may be used to create the longitudinal seal of the oral pouched product as disclosed herein. In such case, the transverse seams(s) may be within the range of from about 2 mm to about 5 mm in width and the longitudinal seam may be within the range of from about 0.5 mm to about 2 mm in width, such as from about 0.5 mm to 1 mm in width.

Still alternatively, ultrasonic welding may be used to create the transverse seal(s) and heat melt-welding may be used to create the longitudinal seal of the oral pouched product as disclosed herein. In such case, the transverse seams(s) may be within the range of from about 0.5 mm to about 2 mm in width, such as from about 0.5 mm to 1 mm, and the longitudinal seam may be within the range of from about 2 mm to about 5 mm in width.

The oral pouched product, such as an oral pouched smokeless tobacco product, as disclosed herein, may comprise a pouch containing a longitudinal fin seam extending along the length of the product, the longitudinal fin seam having a width within the range of from 0.5 to 2 mm, such as from about 0.5 mm to 1 mm. Ultrasonic welding enables the formation of this narrow longitudinal fin seam.

The oral pouched product, such as an oral pouched smokeless tobacco product, as disclosed herein, may comprise a pouch containing at least one transverse fin seam extending along the width of the product, the at least one transverse seam having a width within the range of from 0.5 mm to 2 mm, such as from 0.5 mm to 1 mm. Ultrasonic welding enables the formation of this (these) narrow transverse fin seam(s).

In a specific embodiment of the oral pouched product as disclosed herein, the product has a maximum length of 34 ± 0.5 mm and a maximum width of 9 ± 0.5 mm. This product may include transverse fin seams each one having a width of about 2 to 3 mm. This product may further include a longitudinal fin seam having a width of 2 to 4 mm.

In another specific embodiment of the oral pouched product as disclosed herein, the product has a maximum length of 34 ± 0.5 mm and a maximum width of 9 ± 0.5 mm. This product may include transverse fin seams each one having a width of 2 mm to 3 mm. This product may further include a longitudinal fin seam having a width of 0.5 mm to 1 mm.

In a further specific embodiment of the oral pouched product as disclosed herein, the product has a maximum length of 29 ± 0.5 mm and a maximum width of 9 ± 0.5 mm. This product may include transverse fin seams each one having a width of 0.5 mm to 1 mm. This product may further include a longitudinal fin seam having a width of 2 mm to 4 mm.

In still another specific embodiment of the oral pouched product as disclosed herein, the product has a maximum length of 29 ± 0.5 mm and a maximum width of 9 ± 0.5 mm. This product may include transverse fin seams each one having a width of 0.5 mm to 1 mm. This product may further include a longitudinal fin seam having a width of 0.5 mm to 1 mm.

When making snus according to GothiaTek® standard, which implies hygienic handling of all ingredients and pasteurization of the loaded materials thus assuring a final tobacco composition with negligible levels of bacteria, the typical main ingredients, besides tobacco, are water, sodium chloride (NaCl) and sodium carbonate (Na₂CO₃). Flavours and humectants (e.g. propylene glycol or glycerol) are also common ingredients and additional food approved additives might be used.

There are normally two major steps in the manufacturing process of converting tobacco to a snus composition; a) grinding (or cutting) and sieving and b) snus-processing.

### a) Grinding and sieving

Generally, tobacco flour is produced by batch grinding. Compressed tobacco is emptied from its cases and torn to large fragments which are cut to pieces. The cut tobacco pieces are dried and transported to a mill. The tobacco is ground and ground tobacco particles are sieved and separated into fractions. Too large particles may be brought back to the mill for re-grinding. The cutting, grinding and sieving is done in equipment where foreign objects such as fragments of metallic material and stones are separated and removed from the tobacco. A number of approved fractions are weighed in separate fractions scales. The weighed tobacco flour fractions are collected to pre-set quantities in a container and blended by circulation. The blended tobacco flour is stored in a container. Different types of tobacco flours are kept in separate containers.

### b) Snus-processing

The snus mixture (herein referred to as smokeless tobacco composition) is produced by batch processing and should be carried out in a closed system to minimize the risk of contamination from bacteria or foreign substances. Since automatic feeding of tobacco and the other ingredients is preferred, the whole process may be computer controlled and can be run day and night, all week around.

The process normally starts with loading of tobacco material in powder form (tobacco flour), water, sodium chloride (NaCl) and possibly additional additives, into a cylindrical blender. Loading is done while stirring. The loaded materials are mixed to a homogeneous blend, which is heated by injection of steam. The blend is then kept heated for several hours with support of steam to ensure reduction of the natural bacterial flora in the tobacco and to bring texture, taste and color to the snus blend. Time, temperature and frequency of stirring during heat treatment, parameters specified for different snus blend qualities, are preferably controlled by a process computer program. After heat treatment, the blend is normally chilled by flow of cold water through the blender jacket during stirring. Water, flavours, pH adjuster, such as sodium carbonate, and possibly additional additives may then be added to the chilled blend. The blend is finally mixed to a homogeneous snus composition.

The oral pouched (i.e. portion-packed) products, such as oral pouched smokeless tobacco products, as disclosed herein, may be positioned randomly in a container or in a pattern, as described in WO 2012/069505. Alternatively or additionally, each oral pouched (i.e. portion-packed) product may be placed in a sachet.

The invention will now be illustrated by means of the following non-limiting examples.

### EXAMPLES

### Example 1

A smokeless tobacco composition, having a moisture content of about 56% w/w, suitable for pouch packing using the herein referenced NYPS technology was produced in accordance with the above disclosed GothiaTek® standard.

The tobacco composition was packed in pouches using a pouch packer machine in accordance with the herein referenced NYPS technology.

Pouched snus products with three different sizes, as outlined in Table 1, were produced. The maximum length (L) of the pouch, i.e. the maximum length of the product, was measured along the direction from the first outer side end to the second outer side end of the pouch as shown in Figure 1. The maximum width (W) of the pouch, i.e. the maximum width of the product, was measured in a direction perpendicular to the length direction as shown in Figure 1.

The thickness of the pouched product (in Table 1 referred to as thickness at waist) was about 5 ± 0.5 mm for Example 1, Ref. 1 and Ref. 2.

The target weight per pouched product (i.e. the total weight of pouch and snus) is also shown in Table 1.

The pouched snus products according to Example 1, Ref. 1 and Ref. 2 were non-post-moisturized.

**Table 1**

| | Example 1 | Ref. 1 | Ref. 2 |
|---|---|---|---|
| Target weight per pouched product [g] | 0.5 ± 0.2 | 0.5 ± 0.2 | 0.9 ± 0.2 |
| Total moisture content of pouched product (% w/w) | 52 ± 1 | 52 ± 1 | 52 ± 1 |
| Maximum length of product [mm] | 34 | 27 | 34 |
| Maximum width of product [mm] | 9 | 14 | 18 |
| Ratio of maximum length to maximum width | 3.8 | 1.9 | 1.9 |
| Thickness at waist [mm] | ∼5 | ∼4.5 | ∼5.5 |
| Width at waist [mm] | ∼5 | ∼12 | ∼15 |
| Transverse waist circumference (TWC) [mm] | 21 | 30 | 38 |
| Longitudinal circumference (LC) [mm] | 62 | 43 | 57 |
| LC-to-TWC ratio | 3.0 | 1.4 | 1.5 |
| Width of each transverse seam [mm] | ∼3 | ∼3 | ∼3 |

Each pouched product was weighed and the weight was recorded.

The target weight per pouched product is the total weight of the product (i.e. including filling material and pouch).

The moisture content of Table 1 is the average value of 3 to 4 measurements of about 3 ± 0.5 g of the respective oral pouched smokeless tobacco products.

The maximum length and maximum width of the product were measured as illustrated in Figure 1a.

The approximate "thickness at waist" (generally referred to as the thickness of the product) was measured for each product as the height of the product, in a non-compressed state, at the centre of the maximum length of the product as illustrated in Figure 1b. The centre of the maximum length of the product is herein referred to as the waist of the product.

The approximate "width at waist" was measured in a direction perpendicular to the direction in which "thickness at waist" was measured.

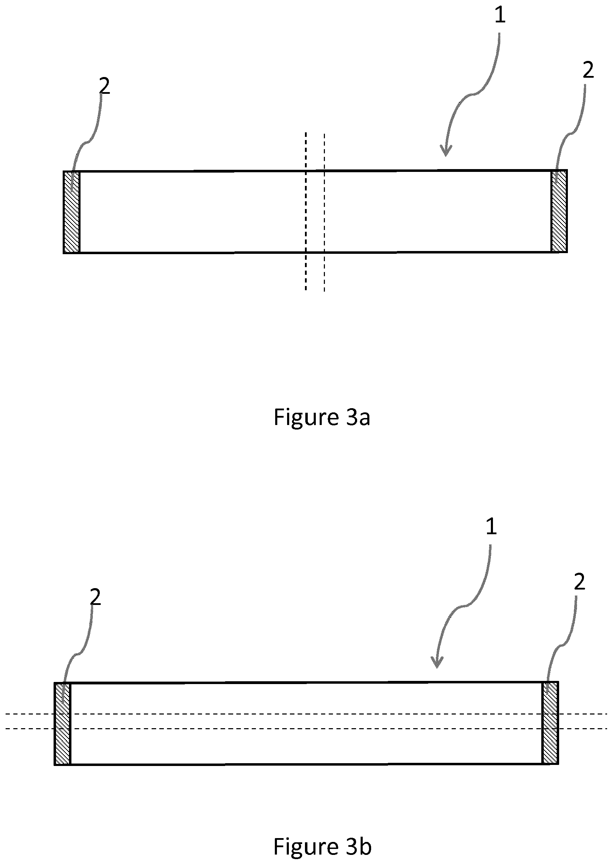

The transverse waist circumference (TWC) of the pouch was measured by cutting the product 1 around the waist of the product, such as along the two dotted lines shown in Figure 3a. The thereby provided continuous annular piece of the pouch was cut open and the length of the resulting rectangular piece of pouch material was measured. This length is herein referred to as the transverse waist circumference of the pouch.

The longitudinal circumference (LC) of the pouch was measured by cutting the product 1 along the length of the product 1, such as along the two dotted lines shown in Figure 3b. The thereby provided continuous annular piece of the pouch was cut open between the transverse seams and the length of the resulting rectangular piece of pouch material was measured. This length, which thus excludes the width of the two transverse seams 2, is herein referred to as the longitudinal circumference of the pouch.

The LC-to-TWC ratio of the product according to Example 1 was about 3.0.

The thickness at waist and the width at waist of the product according to Example 1 was substantially the same; about 5 mm.

### Example 1a: Sensory characteristics

Each individual of a panel of 12 individuals were provided with 4 pouched snus products of Example 1, 4 pouched snus products of Ref. 1 and 4 pouched snus products of Ref. 2. The format of Ref. 1 is, when sold commercially, referred to as "mini". The format of Ref. 2 is, when sold commercially, referred to as "large".

Each pouched snus product was provided in a 14 ml Falcon test tube with a removable sealing cap.

For each type of product, the individuals were instructed to put a snus product in the mouth between the upper lip and gum and keep it there for 5, 10, 15 and 30 minutes, respectively, which represent typical snus usage periods.

After use thereof, the 12 individuals were asked to evaluate the overall experience of the different groups of pouched snus products by rating, on a scale from 1 - 5, where 1 is the lowest ranking and 5 is the highest ranking, whether the products were (i) comfort, (ii) stayed in place, (iii) discreet, and (iv) runny. It should be noted that with regard to runniness, ranking 5 corresponds to a low degree of experienced runniness while ranking 1 corresponds to a high degree of experienced runniness.

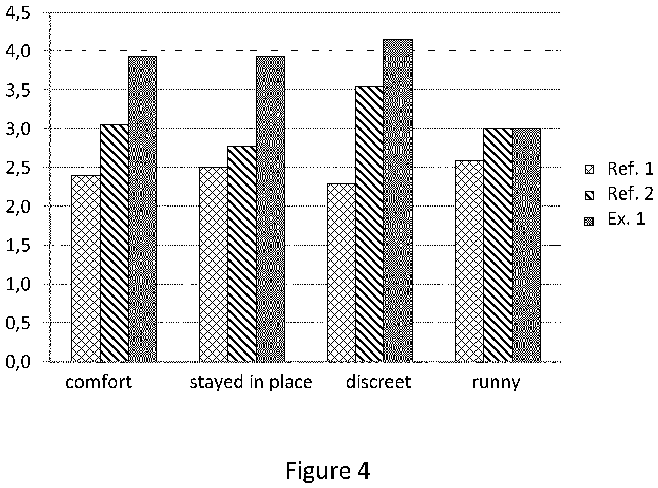

The results (median values) are presented in Figure 4. These results clearly show that the users found the oral pouched snus product of Example 1 better than the reference products in view of comfort and discreetness upon use thereof. The users also experienced that the product of Example 1 stayed better in place in the mouth as compared to the reference products. However, the users did not experience any increased runniness while using the product of Example 1, which is considered to be advantageous.

As shown by this experiment, the non-post-moisturized pouched snus product of Example 1, having a moisture content of about 51 - 53% w/w based on the total weight of the product, was found to fit comfortably and discreetly in the user's mouth and stayed in place in the mouth upon use thereof while still avoiding the feeling of runniness.

### Example 1b: Sensory characteristics

57 snus users normally using pouched snus products having the so-called "slim" format were provided with a can of 24 pouched snus products in accordance with Example 1 to compare with their regular "slim" product.

Data for "slim" products sold by Swedish Match North Europe under the brand name "The LaB Series", such as "The LaB 02" (Ref. 3a), "The LaB 03 Slim White" (Ref. 3b) and "The LaB 22" (Ref 3c), are provided in Table 2. The product "The LaB 02" is post-moisturized and the products "The LaB 03 Slim White" and "The LaB 22" are non-post-moisturized.

Figure 5 illustrates the maximum length (35 mm) and the maximum width (14 mm) of the commercially available pouched snus product "LaB 02" (Ref. 3a) having a product weight of 0.9 g.

Figure 6 illustrates the maximum length (37 mm) and the maximum width (13 mm) of another commercially available pouched snus product called "Camel Frost" having a product weight 0.6 g.

**Table 2**

| | Example 1 | Ref. 3a | Ref. 3b | Ref. 3c |
|---|---|---|---|---|
| Target weight per pouched product [g] | 0.5 ± 0.2 | 0.9 ± 0.2 | 0.9 ± 0.2 | 0.7 ± 0.2 |
| Total moisture content of pouched product (% w/w) | 52 ± 1 | ∼46.5 | ∼50.5 | ∼27.5 |
| Maximum length of product [mm] | 34 | 35 | 34 | 35 |
| Maximum width of product [mm] | 9 | 14 | | |
| Ratio of maximum length to maximum width | 3.8 | 2.5 | 2.4 | 2.5 |

Each individual had to consider the following statements:
a) The product has a good fit under the lip.
b) The product feels comfortable.

The answers for the individuals to choose from were:
1. Applies much more on my normal format (i.e. the "slim" format)
2. Applies somewhat more on my normal format (i.e. the "slim" format)
3. No difference between my regular format (i.e. the "slim" format) and the new format (i.e. Example 1)
4. Applies somewhat more on the new format (i.e. Example 1)
5. Applies much more on the new format (i.e. Example 1)

67% of the individuals responded that statements a) and b) applied somewhat more (i.e. alternative 4) or much more (i.e. alternative 5) on the new format in accordance with Example 1. Thus, a majority of the individuals experienced that the oral pouched snuff product as disclosed herein had a better fit under the lip and was more comfortable than an oral pouched snuff product having the "slim format", such as Ref. 3a-c.

### Example 2: Sensory characteristics

Each individual of a panel, 11 panelists the first day and 12 panelists the second day, was provided with two different types of pouched snus products in two replicates on two days in a row.

The reference product (called Ref. 4) was the commercially available product called "G.3 White XSTR PSWS" (Slim White Portion, 0.9 g, 1.8% nicotine, 46.5% water).

Oral pouched snus products (called Example 2) having the same format as in Example 1 and containing the same snus composition as in the reference product were produced.

For each type of product, the panelists were instructed to put a snus product in the mouth under the upper lip and keep it there for 10 minutes, with a pause of a couple of minutes in between the two tested products.

Each panelist was thereafter asked to rank which type of product that provided the highest perceived nicotine delivery.

In total, there were 23 answers from the test. The test was evaluated as a paired difference test. The results are presented in Table 3.

**Table 3**

| | Ref. 4 | Example 2 |
|---|---|---|
| Target weight per pouched product [g] | 0.9 ± 0.2 | 0.5 ± 0.2 |
| Nicotine per portion (mg) | 16 | 9 |
| Total moisture content of pouched product (% w/w) | ∼46.5 | ∼46.0% |
| Maximum length of product [mm] | 34 | 34 |
| Maximum width of product [mm] | 14 | 9 |
| Ratio of maximum length to maximum width | 2.4 | 3.8 |
| Perceived delivery of nicotine | 10 | 13 |

10 answers stated that the product of Ref. 4 provided a higher perceived nicotine delivery than the product of Example 2.

13 answers stated that the product of Example 2 provided a higher perceived nicotine delivery than the product of Ref. 4.

Thus, despite the smaller format and less weight of the product in accordance with Example 2, the result of the test showed that even more individuals perceived the nicotine delivery as higher from the product of Example 2 despite the lower nicotine content of the product of Example 2 compared to the reference product. This result was unexpected since the amount of nicotine (9 mg) in the product of Example 2 was lower than the amount of nicotine (16 mg) in the product of Ref. 4.

## Claims

1. An oral pouched smokeless tobacco product comprising a filling material and a saliva-permeable pouch enclosing the filling material, the product having a rectangular shape with a maximum length and a maximum width, **characterized in that** the maximum width of the product is within the range of from 5 mm to 10 mm, the maximum length of the product is within the range of from 25 mm to 40 mm, the ratio of maximum length to maximum width is within the range of from 3 to 6, the oral pouched smokeless tobacco product has a weight within the range of from 0.3 g to 0.7 g, the oral pouched smokeless tobacco product has a moisture content within the range of 40 to 60% w/w based on the total weight of the oral pouched smokeless tobacco product, and the product has a thickness in a direction perpendicular to the width of the product, said thickness being within the range of from 2 to 8 mm.

2. An oral pouched smokeless tobacco product according to claim 1, wherein the oral pouched smokeless tobacco product has a moisture content within the range of 40 to 55% w/w based on the total weight of the oral pouched smokeless tobacco product.

3. An oral pouched smokeless tobacco product according to claim 1, wherein the oral pouched smokeless tobacco product has a moisture content within the range of 45 to 55% w/w based on the total weight of the oral pouched smokeless tobacco product.

4. An oral pouched smokeless tobacco product according to any one of the preceding claims, wherein the maximum width of the product is within the range of from 5 mm to 9.5 mm or 7 mm to 9.5 mm.

5. An oral pouched smokeless tobacco product according to any one of the preceding claims, wherein the ratio of maximum length to maximum width is within the range of from 3 to 5.

6. An oral pouched smokeless tobacco product according to any one of the preceding claims, wherein said thickness is within the range of from 3 to 7 mm or from 4 to 6 mm.

7. An oral pouched smokeless tobacco product according to claim 6, wherein the thickness of the product at half the maximum length of the product is within the range of from 4 to 6 mm.

8. An oral pouched smokeless tobacco product according to claim 7, wherein the product has a width at half the maximum length of the product, said width at half the maximum length of the product being within the range of from 4 to 6 mm.

9. An oral pouched smokeless tobacco product according to any one of the preceding claims, wherein the oral pouched product has a dry weight within the range of from 0.2 g to 0.4 g.

10. An oral pouched smokeless tobacco product according to any one of the preceding claims, wherein the filling material comprises tobacco material, such as moist snuff.

11. An oral pouched smokeless tobacco product according to any one of the preceding claims, wherein the oral pouched smokeless tobacco product is non-post-moisturized.

12. An oral pouched smokeless tobacco product according to claim 11, wherein the oral pouched smokeless tobacco product has moisture content within the range of from 45 to 55% w/w, such as within the range of from 50 to 53% w/w, based on the total weight of the product.

13. An oral pouched smokeless tobacco product according to any of the preceding claims, wherein the pouch contains a longitudinal fin seam extending along the length of the product, said longitudinal seam having a width within the range of from 0.5 mm to 2 mm, such as from 0.5 mm to 1 mm.

14. An oral pouched smokeless tobacco product according to any of the preceding claims, wherein the pouch contains at least one transverse fin seam extending along the width of the product, said at least one transverse seam having a width within the range of from 0.5 mm to 2mm, such as from 0.5 mm to 1 mm.

## Patentansprüche

1. Orales im Beutel verpacktes rauchfreies Tabakprodukt, umfassend ein Füllmaterial und einen speicheldurchlässigen Beutel, der das Füllmaterial umschließt, wobei das Produkt eine rechteckige Form mit einer maximalen Länge und einer maximalen Breite aufweist, **dadurch gekennzeichnet, dass** die maximale Breite des Produkts im Bereich von 5 mm bis 10 mm liegt, die maximale Länge des Produkts im Bereich von 25 mm bis 40 mm liegt, das Verhältnis der maximalen Länge zur maximalen Breite im Bereich von 3 bis 6 liegt, das orale im Beutel verpackte rauchfreie Tabakprodukt ein Gewicht im Bereich von 0,3 g bis 0,7 g aufweist, das orale im Beutel verpackte rauchfreie Tabakprodukt einen Feuchtigkeitsgehalt im Bereich von 40 bis 60 Gew.-%/Gew.-%, basierend auf dem Gesamtgewicht des oralen im Beutel verpackten rauchfreien Tabakprodukts, aufweist, und das Produkt eine Dicke in einer Richtung senkrecht zur Breite des Produkts aufweist, wobei die Dicke im Bereich von 2 bis 8 mm liegt.

2. Orales im Beutel verpacktes rauchfreies Tabakprodukt nach Anspruch 1, wobei das orale im Beutel verpackte rauchfreie Tabakprodukt einen Feuchtigkeitsgehalt im Bereich von 40 bis 55 Gew.-%/Gew.-%, basierend auf dem Gesamtgewicht des oralen im Beutel verpackten rauchfreien Tabakprodukts, aufweist.

3. Orales im Beutel verpacktes rauchfreies Tabakprodukt nach Anspruch 1, wobei das orale im Beutel verpackte rauchfreie Tabakprodukt einen Feuchtigkeitsgehalt im Bereich von 45 bis 55 Gew.-%/Gew.-%, basierend auf dem Gesamtgewicht des oralen im Beutel verpackten rauchfreien Tabakprodukts, aufweist.

4. Orales im Beutel verpacktes rauchfreies Tabakprodukt nach einem der vorstehenden Ansprüche, wobei die maximale Breite des Produkts im Bereich von 5 mm bis 9,5 mm oder 7 mm bis 9,5 mm liegt.

5. Orales im Beutel verpacktes rauchfreies Tabakprodukt nach einem der vorstehenden Ansprüche, wobei das Verhältnis von maximaler Länge zur maximalen Breite im Bereich von 3 bis 5 liegt.

6. Orales im Beutel verpacktes rauchfreies Tabakprodukt nach einem der vorstehenden Ansprüche, wobei die Dicke im Bereich von 3 bis 7 mm oder von 4 bis 6 mm liegt.

7. Orales im Beutel verpacktes rauchfreies Tabakprodukt nach Anspruch 6, wobei die Dicke des Produkts bei halber maximaler Länge des Produkts im Bereich von 4 bis 6 mm liegt.

8. Orales im Beutel verpacktes rauchfreies Tabakprodukt nach Anspruch 7, wobei das Produkt eine Breite bei halber maximaler Länge des Produkts aufweist, wobei die Breite bei halber maximaler Länge des Produkts im Bereich von 4 bis 6 mm liegt.

9. Orales im Beutel verpacktes rauchfreies Tabakprodukt nach einem der vorstehenden Ansprüche, wobei das orale im Beutel verpackte Produkt ein Trockengewicht im Bereich von 0,2 g bis 0,4 g aufweist.

10. Orales im Beutel verpacktes rauchfreies Tabakprodukt nach einem der vorstehenden Ansprüche, wobei das Füllmaterial Tabakmaterial, wie feuchten Schnupftabak, umfasst.

11. Orales im Beutel verpacktes rauchfreies Tabakprodukt nach einem der vorstehenden Ansprüche, wobei das orale im Beutel verpackte rauchfreie Tabakprodukt nicht nach befeuchtet ist.

12. Orales im Beutel verpacktes rauchfreies Tabakprodukt nach Anspruch 11, wobei das orale im Beutel verpackte rauchfreie Tabakprodukt einen Feuchtigkeitsgehalt im Bereich von 45 bis 55 Gew.-%/Gew.-%, wie im Bereich von 50 bis 53 Gew.-%/Gew.-%, basierend auf dem Gesamtgewicht des Produkts, aufweist.

13. Orales im Beutel verpacktes rauchfreies Tabakprodukt nach einem der vorstehenden Ansprüche, wobei der Beutel eine Längsrippennaht enthält, die sich über die Länge des Produkts erstreckt, wobei die Längsnaht eine Breite im Bereich von 0,5 mm bis 2 mm, wie 0,5 mm bis 1 mm, aufweist.

14. Orales im Beutel verpacktes rauchfreies Tabakprodukt nach einem der vorstehenden Ansprüche, wobei der Beutel mindestens eine Querrippennaht enthält, die sich entlang der Breite des Produkts erstreckt, wobei die mindestens eine Quernaht eine Breite im Bereich von 0,5 mm bis 2 mm, wie 0,5 mm bis 1 mm, aufweist.

## Revendications

1. Produit de tabac sans fumée emballé à usage oral comprenant un matériau de remplissage et une poche perméable à la salive renfermant le matériau de remplissage, le produit ayant une forme rectangulaire et une longueur maximale et une largeur maximale, **caractérisé en ce que** la largeur maximale du produit est comprise entre 5 mm et 10 mm, la longueur maximale du produit est comprise entre 25 mm et 40 mm, le ratio de la longueur maximale par rapport à la largeur maximale est compris entre 3 et 6, le produit de tabac sans fumée emballé à usage oral a un poids compris entre 0,3 g et 0,7 g, le produit de tabac sans fumée emballé à usage oral a une teneur en humidité comprise entre 40 et 60 % poids/poids par rapport au poids total du produit oral de tabac sans fumée emballé, et le produit a une épaisseur dans une direction perpendiculaire à la largeur du produit, ladite épaisseur étant comprise entre 2 et 8 mm.

2. Produit de tabac sans fumée emballé à usage oral selon la revendication 1, dans lequel le produit de tabac sans fumée emballé à usage oral a une teneur en humidité comprise entre 40 et 55 % poids/poids par rapport au poids total du produit oral de tabac sans fumée emballé.

3. Produit de tabac sans fumée emballé à usage oral selon la revendication 1, dans lequel le produit de tabac sans fumée emballé à usage oral a une teneur en humidité comprise entre 45 et 55 % poids/poids par rapport au poids total du produit oral de tabac sans fumée emballé.

4. Produit de tabac sans fumée emballé à usage oral selon l'une quelconque des revendications précédentes, dans lequel la largeur maximale du produit est comprise entre 5 mm et 9,5 mm ou entre 7 mm et 9,5 mm.

5. Produit de tabac sans fumée emballé à usage oral selon l'une quelconque des revendications précédentes, dans lequel le ratio de la longueur maximale par rapport à la largeur maximale est compris entre 3 et 5.

6. Produit de tabac sans fumée emballé à usage oral selon l'une quelconque des revendications précédentes, dans lequel ladite épaisseur est comprise entre 3 et 7 mm ou entre 4 et 6 mm.

7. Produit de tabac sans fumée emballé à usage oral selon la revendication 6, dans lequel l'épaisseur du produit à la moitié de la longueur maximale du produit est comprise entre de 4 à 6 mm.

8. Produit de tabac sans fumée emballé à usage oral selon la revendication 7, dans lequel le produit a une largeur à la moitié de la longueur maximale du produit, ladite largeur à la moitié de la longueur maximale étant comprise entre 4 et 6 mm.

9. Produit de tabac sans fumée emballé à usage oral selon l'une quelconque des revendications précédentes, dans lequel le produit oral emballé a un poids sec compris entre 0,2 g et 0,4 g.

10. Produit de tabac sans fumée emballé à usage oral selon l'une quelconque des revendications précédentes, dans lequel le matériau de remplissage comprend un matériau de tabac, tel que du tabac à priser humide.

11. Produit de tabac sans fumée emballé à usage oral selon l'une quelconque des revendications précédentes, dans lequel le produit de tabac sans fumée à usage oral n'est pas post-humidifié.

12. Produit de tabac sans fumée emballé à usage oral selon la revendication 11, dans lequel le produit de tabac sans fumée emballé à usage oral a une teneur en humidité comprise entre 45 et 55 % poids/poids, notamment de l'ordre de 50 à 53 % poids/poids, par rapport au poids total du produit.

13. Produit à base de tabac sans fumée emballé à usage oral selon l'une quelconque des revendications précédentes, dans lequel la poche contient une couture à bords repliés longitudinale s'étendant sur la longueur du produit, ladite couture longitudinale ayant une largeur comprise entre 0,5 mm et 2 mm, notamment comprise entre 0,5 mm et 1 mm.

14. Produit de tabac sans fumée emballé à usage oral selon l'une quelconque des revendications, dans lequel la poche contient au moins une couture à bords repliés transversale ayant une largeur comprise entre 0,5 mm et 2 mm, notamment entre 0,5 mm et 1 mm.
